# EUROPÄISCHE PATENTSCHRIFT

(11) **EP 0 394 985 B1**
(45) Veröffentlichungstag und Bekanntmachung des Hinweises auf die Patenterteilung: **07.04.1993**
(21) Anmeldenummer: 90107816.2
(22) Anmeldetag: 25.04.1990
(51) Int. Cl.: C07C 205/57, C07C 201/12

(54) **Verfahren zur Herstellung von Nitro-benzoesäure-alkylestern**
Process for preparing nitro benzoic acid alkyl esters
Procédé pour la préparation d'esters alkyliques d'acides nitrobenzoiques

(30) Priorität: 26.04.1989 DE 3913781
(43) Veröffentlichungstag der Anmeldung: 31.10.1990
(73) Patentinhaber: HOECHST AKTIENGESELLSCHAFT, 65926 Frankfurt am Main (DE)
(72) Erfinder: Papenfuhs, Theodor, Dr., D-6000 Frankfurt am Main (DE); Hess, Reiner, Dr., D-6200 Wiesbaden (DE); Fuss, Andreas, Dr., D-8757 Karlstein (DE)

(56) Entgegenhaltungen:
- EP-A- 0 003 205
- DE-A- 2 317 276
- DE-C- 3 335 312
- CHEMICAL ABSTRACTS, Band 82, Nr. 23, 9.Juni 1975 Columbus, Ohio, USA; S.A.ELGAZIN et al. "Ethyl p-nitrobenzoate" Seite 564, Spalte 1, Zusammenfassung-Nr. 155 862z

## Beschreibung

Die vorliegende Erfindung betrifft ein gegenüber dem Stand der Technik einfaches und vorteilhaftes Verfahren zur Herstellung von Nitro-benzoesäure-alkylestern durch Veresterung von Nitro-benzoesäuren mit niederen Alkanolen in Gegenwart von Polyfluoralkansulfonsäuren oder deren Hydrat als Katalysator.

Nitro-benzoesäure-alkylester werden im allgemeinen analog dem nachstehenden Reaktionsschema 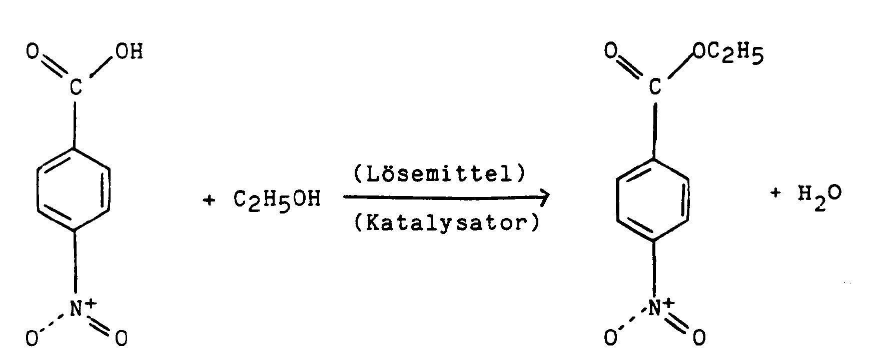
welches die Darstellung des 4-Nitro-benzoesäure-ethylesters zeigt, d.h. durch Behandeln der Nitro-benzoesäure mit Ethanol in Gegenwart von Schwefelsäure, hergestellt (B. 71 (1938), 335, 338 oder J.Chin. chem.Soc. 13 (1946), 22, 24 oder M. 66 (1935) 316, 326). Die hierbei erzielten Reinheiten und Ausbeuten sind mäßig, da der Umsatz nur durch den wasserentziehenden Effekt der Schwefelsäure zustande kommt, die aber gleichzeitig zu verschiedenen Nebenreaktionen, wie Sulfonierung und Oxidation, Anlaß gibt und deshalb als "Lösungsmittel" für die Veresterung ungeeignet ist.

Weitere Möglichkeiten der Veresterung sind das Arbeiten mit Dialkylsulfaten als O-Alkylierungsmittel in Gegenwart von basischen Katalysatoren, wie Dicyclohexylamin (Stodola, J. Org. Chem. 29 (1964), Nr. 8, 2490-91), oder mit Diazoalkanen (J. Labelled Compd. Radiopharm. 1983, 20 (9), 1047-59). Beide Möglichkeiten sind jedoch wegen der extremen Toxizität der eingesetzten Alkylierungsmittel nur mit sehr hohem sicherheitstechnischen Aufwand im Produktionsmaßstab realisierbar.

Es gibt ferner eine große Zahl von Alkylierungsmitteln, deren mangelnde technische Verfügbarkeit eine Anwendung im Produktionsmaßstab verhindern:
Alkylphosphit-diethylazodicarboxylat (Pol.J. Chem. 1982, 56 (1), 149-52),
Alkoxy- und Alkyl-thiomethyleniminium-Salze (Yukagaku 1984, 33 (11), 776-9),
Trialkyl-oxonium-Salze (J. Org.Chem. 44 (1979) 7, 1149).

Einen Überblick über die verschiedenen Alkylierungsmittel für den Labormaßstab vermittelt der Band "Carbonsäure-Derivate" (HOUBEN-WEYL, "Methoden der Organischen Chemie", Band E 5, Teil 1, Seiten 659 ff.), in dem unter anderem auch Tetraalkyloxy-Silane bzw. Chlor-Silane und verschiedene Derivate der Phosphorsäure als O-Alkylierungsagenzien für Carbonsäuren aufgeführt sind. Allen dort aufgeführten Reagenzien gemeinsam ist die komplexe, teilweise nur im Labormaßstab auf die geschilderte Weise durchführbare Herstellungsweise und die erhebliche Toxizität hochreaktiver Alkylierungsmittel (wie beispielsweise Diazomethan). So ist denn auch keine Anmeldung, die den Einsatz der geschilderten Alkylierungsmittel für die Synthese von Nitro-benzoesäure-estern beschreibt, zu finden.

Als technisches Herstellungsverfahren für beispielsweise 4-Nitro-benzoesäure-methylester wird die Umsetzung von 4-Nitro-benzoesäure mit 1400 mol-% Methanol bei 190°C in zwei Verfahrensschritten beschrieben (DE 33 35 312-C): Hierbei wird zunächst mit der halben Menge Alkohol unter Druck erhitzt, dann abgekühlt und entspannt, anschließend das entstandene Wasser - zusammen mit Methanol - abdestilliert, erneut Methanol zugegeben und unter Druck nochmals auf 190°C erhitzt. Dieses Verfahren ergibt trotz großen verfahrenstechnischen Aufwandes (Autoklav; Stufenzahl) nur eine Ausbeute von 93 % der Theorie, wobei besonders die durch den hohen Methanolüberschuß mäßige Raumausbeute und die durch die vielen Verfahrensschritte sehr mäßige Raum-Zeit-Ausbeute zu bemängeln sind.

In einer anderen Offenlegungsschrift (SU 455 943) wird die Herstellung von 4-Nitro-benzoesäure-ethylester wie folgt beschrieben:
4-Nitro-benzoesäure wird mit 370 mol-% Ethanol in Gegenwart von 79 mol-% Ammoniumhydrogensulfat - also einer fast äquimolaren Menge - bei 80°C am Rückfluß gehalten. Die Aufarbeitung erfolgt über eine nicht näher beschriebene Phasentrennung und Kristallisation aus Ethanol. Als Ausbeute werden bei diesem Verfahren 95,5 % der Theorie angegeben. Von einer Möglichkeit der Rückführung der den - in fast molarer Menge - zugesetzten Katalysator enthaltenden Unterphase ist nicht die Rede; auch die Qualität des Endproduktes ist nicht charakterisiert.

Beim Arbeiten mit Ammoniumhydrogensulfat gilt das weiter oben für die Umsetzung mit Schwefelsäure Gesagte: Qualität und Aspekt des Produktes sind wegen Nebenreaktionen mit Schwefelsäure bzw. Hydrogensulfat nur mäßig. Dazu kommt die im Vergleich mit verdünnter Schwefelsäure nur unwesentlich geringere Korrosivität des Hydrogensulfats in Verdünnung, die ein Arbeiten in korrosionsfesten Materialien (Hastelloy o.ä.) erforderlich macht.

Es bestand somit ein Bedarf für ein technisches Verfahren zur Herstellung von Nitro-benzoesäure-estern niedriger Alkanole (R-OH mit R = CH₃, C₂H₅ oder C₃H₇), das mit einem Katalysator arbeitet, der in "echt" katalytischen Mengen zugesetzt wird und möglichst ohne Regenerierung zurückgeführt werden kann und nicht, wie beispielsweise die Schwefelsäure, Anlaß zu Nebenreaktionen mit Ausgangsverbindungen oder Reaktionsprodukt gibt.

Es wurde nun gefunden, daß man Nitro-benzoesäure-alkyl(C₁-C₃)-ester auf einfache und vorteilhafte Weise herstellen kann, indem man die zu veresternde Nitro-benzoesäure mit einem Überschuß von etwa 300 bis etwa 600 mol-%, vorzugsweise etwa 400 bis etwa 500 mol-% eines Alkanols(C₁-C₃) in einem gegenüber den Ausgangsverbindungen und dem Reaktionsprodukt inerten Lösungsmittel in Gegenwart einer Polyfluoralkansulfonsäure der allgemeinen Formel (1)

Y(CₙF₂ₙ)SO₃H (1)

in welcher Y ein Wasserstoff- oder Fluoratom bedeutet, mit der Maßgabe, daß im Falle Y = H dieses Wasserstoffatom in β-Stellung zur Sulfonsäuregruppe steht, oder deren Hydrat als Katalysator in einer Menge von etwa 0,1 bis etwa 20 mol-%, vorzugsweise etwa 2 bis etwa 10 mol-%, besonders vorteilhaft etwa 2 bis etwa 4 mol-%, bezogen auf eingesetzte Nitro-benzoesäure, bei Temperaturen von etwa 60 bis etwa 120°C, vorzugsweise etwa 62 bis etwa 108°C, besonders bevorzugt von etwa 62 bis etwa 95°C, umsetzt.

Der beim erfindungsgemäßen Verfahren zur Anwendung gelangende Katalysator der genannten Formel (1), beispielsweise die Tetrafluorethansulfonsäure oder Hexafluorpropansulfonsäure bzw. deren Monohydrate, erfüllt voll und ganz die gestellten Bedingungen, indem beim vorliegenden Verfahren keine auf den Katalysator zurückzuführenden Nebenreaktionen auftreten und der Katalysator selbst durch einfache wäßrige Extraktion aus der angefallenen Reaktionslösung zurückgewonnen und ohne weitere Regenerierung in den Folgeansatz zurückgeführt werden kann.

Als zu veresternde Nitro-benzoesäure kann die 2-Nitro-, 3-Nitro- und 4-Nitro-benzoesäure eingesetzt werden.

Geeignete inerte Lösungsmittel sind beispielsweise Benzol, Toluol, Xylole, Chlorbenzol, Ethylbenzol, Tetrachlorkohlenstoff, Chloroform, Cyclohexan, 1,2-Dichlor-ethan, Isopropylether, Methylbutylether, Methylbutylketon, Methylethylketon oder Propylisopropylether.

Im Einzelnen wird das erfindungsgemäße Verfahren wie folgt durchgeführt:
Die zu veresternde Nitro-benzoesäure wird, gelöst im inerten Lösungsmittel, vorgelegt und die Polyfluoralkansulfonsäure als solche oder in Form des Hydrats (Katalysator) in einer Menge von etwa 0,1 bis etwa 20 mol-%, vorzugsweise etwa 2 bis etwa 10 mol-%, besonders vorteilhaft etwa 2 bis etwa 4 mol-%, bezogen auf den molaren Einsatz der Nitro-benzoesäure, zugesetzt. Die Lösung wird auf etwa 60 - etwa 120°C, vorzugsweise etwa 62 - etwa 108°C aufgeheizt. Dann wird mit der Zugabe von 300 - 600 mol-%, vorzugsweise 400 - 500 mol-% Alkanol (ROH mit R = CH₃, C₂H₅ oder C₃H₇), begonnen, die derartig erfolgt, daß die Sumpftemperatur der Reaktionsmischung nicht unter etwa 60°C absinkt und nicht über etwa 105°C ansteigt. Hierbei wird kontinuierlich ein Gemisch aus dem zugegebenen Alkanol, dem inerten Lösungsmittel und dem bei der Reaktion entstehenden Wasser abdestilliert.

Nach beendeter Zugabe des Alkanols und nach einer entsprechenden Phase der Nachreaktion steigt die Übergangstemperatur der Destillation an, wodurch angezeigt wird, daß die Veresterung beendet ist. Dann wird noch ein Gemisch aus dem Alkanol und dem Lösungsmittel abdestilliert und schließlich durch Rühren auf Raumtemperatur abgekühlt.

Die Umsetzung (Veresterung) kann bei normalem, vermindertem oder überhöhtem Druck vorgenommen werden.

Die Extraktion des Katalysators zur erneuten Anwendung in den Folgeansätzen erfolgt in 1 - 5, vorzugsweise 2 - 3 Extraktionsschritten mit jeweils 1 - 20 Vol.-%, vorzugsweise 1,5 - 3 Vol.-% Wasser, bezogen auf das Volumen des Reaktionsansatzes.

Es folgt ein Waschzyklus mit wäßrigem Alkali zur Entfernung von geringen Spuren nichtumgesetzter Nitro-benzoesäure. Anschließend wird das Lösungsmittel, gegebenenfalls mit Wasserdampf, abdestilliert und der Nitrobenzoesäure-ester als Schmelze (Unterphase) abgetrennt.

In den nachstehenden Beispielen wird an einzelnen Nitro-benzoesäure-estern verdeutlicht, daß die Umsetzung einfach durchzuführen ist. Hervorzuheben ist nochmals, daß die Katalysatorextrakte ohne weitere Aufarbeitung in den Folgeansatz zurückgeführt werden können, wobei dann im Verlauf des Ansatzes das Extrakt-Wasser abdestilliert wird.

### Beispiel 1

In einem 2-l-Vierhalskolben mit Rührer, Innenthermometer, innentemperaturgeregeltem Zupump und Destillationsaufsatz werden 350 g (2,1 mol) 3-Nitro-benzoesäure, 700 g Toluol und 13,6 g Hexafluorpropansulfonsäure-Hydrat (0,0544 mol = 2,6 mol-%, bezogen auf 3-Nitro-benzoesäure) eingewogen und unter Rühren auf 95°C erhitzt. Man beginnt dann mit der temperaturgesteuerten Zugabe von 188 g (4,0 mol) Ethanol, wobei sofort nach Beginn der Zugabe ein ternäres Gemisch aus Ethanol, Wasser und Toluol abdestilliert, das bei Abkühlen in zwei Phasen zerfällt, von denen eine hauptsächlich Wasser, die andere hauptsächlich Ethanol enthält. Die Ethanolzugabe wird derartig gesteuert, daß innentemperaturabhängig bei Unterschreitung von 95°C die Zugabe eingestellt wird, um das Reaktionsgemisch nicht an Ethanol zu übersättigen.

Nach einer Zugabedauer von 8 Stunden ist die Reaktion zu Ende geführt und man hat insgesamt 92 g ternäres Gemisch abdestilliert (78 g Unterphase und 14 g Oberphase).

In der Folge werden 149 g eines binären Gemisches aus Ethanol und Toluol abdestilliert, wobei man bei Sumpftemperaturen bis 105°C im schwachen Vakuum arbeitet.

Man wäscht nun dreimal mit jeweils 50 ml Wasser und erhält insgesamt 148 g Waschlauge, die Hexafluorpropansulfonsäure-Hydrat enthält und direkt in den Folgeansatz rückgeführt wird.

In der Folge wird dreimal mit jeweils 100 ml 5 %iger Natronlauge gewaschen, um nichtumgesetzte Nitro-benzoesäure zu entfernen. Man erhält 325 g bzw. 315 ml Waschlauge, aus der die 3-Nitro-benzoesäure durch Ansäuern und Absaugen wiedergewonnen wird.

Die toluolische Produktlösung wird nun in 1000 ml Wasser eingetragen und das Lösemittel Toluol mit Wasserdampf abdestilliert. Man erhält 694 g Toluol, das in den Folgeansatz rückgeführt werden kann.

Man trennt das Produkt als Unterphase bei 60°C über einen Scheidetrichter ab und erhält 397 g 3-Nitro-benzoesäure-ethylester, feucht, der durch Andestillieren bis 120°C bei 100 mbar getrocknet wird. Man erhält eine Ausbeute von 387 g 3-Nitro-benzoesäure-ethylester, trocken, entsprechend einer Ausbeute von 94,7 % der Theorie, mit einem Fließpunkt von 39,7°C und einer Reinheit von 98,6 % nach HPLC.

Verwendet man anstelle der 700 g Toluol die gleiche Menge Benzol, ein Xylol, Chlorbenzol oder Ethylbenzol und verfährt im übrigen wie im vorstehenden Beispiel beschrieben, so gelangt man jeweils zum gleichen Ergebnis.

### Beispiel 2

Man legt in einem 2-l-Vierhalskolben mit der in Beispiel 1 genannten Ausstattung 350 g 2-Nitro-benzoesäure (2,1 mol), 700 g Toluol und 10 g Hexafluorpropansulfonsäure-Hydrat vor, heizt auf 95°C auf und gibt nach dem Vorgehen von Beispiel 1 188 g Ethanol (4,2 mol) zu.

Bei gleicher Aufarbeitung wie in Beispiel 1 erhält man hier 380 g 2-Nitro-benzoesäure-ethylester, feucht, bzw. nach Trocknung 376 g 2-Nitro-benzoesäure-ethylester, trocken, entsprechend einer Ausbeute von 92 % der Theorie, mit einem Fließpunkt von 29,8°C und einer Reinheit von 96,9 % nach HPLC.

### Beispiel 3

In einem 2-l-Vierhalskolben, mit der Ausstattung wie in Beispiel 1 beschrieben, werden eingewogen 700 g Toluol, 190 g Ethanol, 350 g 4-Nitro-benzoesäure und 7 g Tetrafluor-ethansulfonsäure-Hydrat. Dann verfährt man wie in Beispiel 1 beschrieben. Nach der in Beispiel 1 beschriebenen Aufarbeitung erhält man 368 g 4-Nitro-benzoesäureethylester, feucht, bzw. nach Trocknung 361 g 4-Nitrobenzoesäure-ethylester, trocken, entsprechend einer Ausbeute von 88,3 % der Theorie, in einer Reinheit von 98,9 % nach HPLC.

### Beispiel 4

In einem 2-l-Vierhalskolben, mit der Ausstattung wie in Beispiel 1 beschrieben, werden eingewogen 700 g Toluol, 235 g Ethanol (5,2 mol), 350 g 4-Nitro-benzoesäure und 10 g Hexafluorpropansulfonsäure-Hydrat. Anschließend verfährt man wie in Beispiel 1 beschrieben. Nach Aufarbeitung gemäß Beispiel 1 erhält man 386 g 4-Nitro-benzoesäure-ethylester, feucht und nach Trocknung 382 g 4-Nitro-benzoesäure-ethylester, trocken, entsprechend einer Ausbeute von 94,4 % der Theorie, in einer Reinheit von 99,2 % nach HPLC mit einem Erstarrungspunkt von 56°C.

### Beispiel 5

Man legt in einem 2-l-Vierhalskolben mit der in Beispiel 1 genannten Ausstattung 700 g Cyclohexan, 350 g 4-Nitro-benzoesäure (2,1 mol) und 10 g Hexafluorpropansulfonsäure-Hydrat vor, heizt auf 80°C auf und beginnt bei dieser Temperatur mit der Zugabe von 230 g (5,0 mol) Ethanol. Das weitere Vorgehen sowie die Aufarbeitung erfolgen analog Beispiel 1. Man erhält hier 399 g 4-Nitro-benzoesäure-ethylester, feucht, bzw. nach Trocknung durch Andestillieren 383 g, entsprechend 94,5 % der Theorie, 4-Nitro-benzoesäure-ethylester trocken, vom Schmelzpunkt 55,8°C in 98,2 %iger Reinheit nach HPLC.

Verwendet man anstelle der 700 g Cyclohexan die gleiche Menge Tetrachlorkohlenstoff, Chloroform, Cyclohexan, 1,2-Dichlor-ethan, Isopropylether, Methylbutylether, Methylbutylketon, Methylethylketon oder Propylisopropylether und verfährt im übrigen wie im vorstehenden Beispiel beschrieben, so gelangt man jeweils zum gleichen Ergebnis.

## Patentansprüche

1. Verfahren zur Herstellung von Nitro-benzoesäure-alkyl(C₁-C₃)-estern, dadurch gekennzeichnet, daß man die zu veresternde Nitro-benzoesäure mit einem Überschuß von etwa 300 bis etwa 600 mol-% eines Alkanols(C₁-C₃) in einem gegenüber den Ausgangsverbindungen und dem Reaktionsprodukt inerten Lösungsmittel in Gegenwart einer Polyfluoralkansulfonsäure der allgemeinen Formel (1)
Y(CₙF₂ₙ)SO₃H (1)
in welcher Y ein Wasserstoff- oder Fluoratom bedeutet, mit der Maßgabe, daß im Falle Y = H dieses Wasserstoffatom in β-Stellung zur Sulfonsäuregruppe steht, oder deren Hydrat als Katalysator in einer Menge von etwa 0,1 bis etwa 20 mol-%, bezogen auf eingesetzte Nitro-benzoesäure, bei Temperaturen von etwa 60 bis etwa 120°C umsetzt.

2. Verfahren nach Anspruch 1, dadurch gekennzeichnet, daß man bei Temperaturen von etwa 62 bis etwa 108°C umsetzt.

3. Verfahren nach mindestens einem der Ansprüchen 1 und 2, dadurch gekennzeichnet, daß man in Gegenwart von Tetrafluorethan-sulfonsäure oder Hexafluorpropansulfonsäure oder deren Hydraten umsetzt.

4. Verfahren nach mindestens einem der Ansprüche 1 bis 3, dadurch gekennzeichnet, daß man mit einem Überschuß von etwa 400 bis etwa 500 mol-% Alkanol(C₁-C₃) umsetzt.

5. Verfahren nach mindestens einem der Ansprüche 1 bis 4, dadurch gekennzeichnet, daß man in Gegenwart von etwa 2 bis etwa 10 mol-% einer Polyfluoralkansulfonsäure der in Anspruch 1 genannten allgemeinen Formel (1) oder deren Hydrat umsetzt.

6. Verfahren nach mindestens einem der Ansprüche 1 bis 5, dadurch gekennzeichnet, daß man 2-Nitro-, 3-Nitro- oder 4-Nitrobenzoesäure umsetzt.

7. Verfahren nach mindestens einem der Ansprüche 1 bis 6, dadurch gekennzeichnet, daß man in Benzol, Toluol, einem Xylol, Chlorbenzol, Ethylbenzol, Tetrachlorkohlenstoff, Chloroform, Cyclohexan, 1,2-Dichlor-ethan, Isopropylether, Methylbutylether, Methylbutylketon, Methylethylketon oder Propylisopropylether als inertem Lösungsmittel umsetzt.

8. Verfahren nach mindestens einem der Ansprüche 1 bis 7, dadurch gekennzeichnet, daß man die Umsetzung bei vermindertem Druck vornimmt.

9. Verfahren nach mindestens einem der Ansprüche 1 bis 7, dadurch gekennzeichnet, daß man die Umsetzung bei erhöhtem Druck vornimmt.

10. Verfahren nach mindestens einem der Ansprüche 1 bis 9, dadurch gekennzeichnet, daß man das überschüssige Alkanol(C₁-C₃) der vorgelegten, in dem inerten Lösungsmittel gelösten Nitro-benzoesäure, deren Lösung auch den Katalysator enthält, bei Temperaturen von etwa 62 bis etwa 108°C zusetzt.

## Claims

1. A process for the preparation of alkyl (C₁-C₃) nitrobenzoates, which comprises reacting the nitrobenzoic acid to be esterified with an excess of about 300 to about 600 mol % of an alkanol (C₁-C₃) in a solvent which is inert towards the starting compounds and the reaction product in the presence of a polyfluoroalkanesulfonic acid of the general formula (1)
Y(CₙF₂ₙ)SO₃H (1)
in which Y is a hydrogen or fluorine atom, with the proviso that, if Y is H, this hydrogen atom is in the β-position relative to the sulfo group, or the hydrate thereof as catalyst in an amount of about 0.1 to about 20 mol %, relative to the nitrobenzoic acid used, at temperatures from about 60 to about 120°C.

2. The process as claimed in claim 1, wherein the reaction is carried out at temperatures from about 62 to about 108°C.

3. The process as claimed in at least one of claims 1 and 2, wherein the reaction is carried out in the presence of tetrafluoroethanesulfonic acid or hexafluoropropanesulfonic acid or their hydrates.

4. The process as claimed in at least one of claims 1 to 3, wherein the reaction is carried out with an excess of about 400 to about 500 mol % of alkanol (C₁-C₃).

5. The process as claimed in at least one of claims 1 to 4, wherein the reaction is carried out in the presence of about 2 to about 10 mol % of a polyfluoroalkanesulfonic acid of the general formula (1) mentioned in claim 1 or its hydrate.

6. The process as claimed in at least one of claims 1 to 5, wherein 2-nitro-, 3-nitro-, or 4-nitrobenzoic acid is reacted.

7. The process as claimed in at least one of claims 1 to 6, wherein the reaction is carried out in benzene, toluene, xylene, chlorobenzene, ethylbenzene, carbon tetrachloride, chloroform, cyclohexane, 1,2-dichloro-ethane, isopropyl ether, methyl butyl ether, methyl butyl ketone, methyl ethyl ketone or propyl isopropyl ether as the inert solvent.

8. The process as claimed in at least one of claims 1 to 7, wherein the reaction is carried out under reduced pressure.

9. The process as claimed in at least one of claims 1 to 7, wherein the reaction is carried out at superatmospheric pressure.

10. The process as claimed in at least one of claims 1 to 9, wherein the excess alkanol (C₁-C₃) is added to the initially introduced nitrobenzoic acid which is dissolved in the inert solvent, the solution of which also contains the catalyst, at temperatures from about 62 to about 108°C.

## Revendications

1. Procédé de préparation de nitrobenzoates d'alkyles en C₁-C₃ caractérisé en ce que l'on fait réagir l'acide nitrobenzoïque à estérifier avec un excès d'environ 300 à 600 mol-% d'un alcanol en C₁-C₃, dans un solvant chimiquement inerte à l'égard des composés dont on part et du produit formé et en présence comme catalyseur d'un acide polyfluoralcane-sulfonique de formule générale (1) :
Y (CₙF₂ₙ)SO₃H (1)
(dans laquelle Y désigne un atome d'hydrogène ou de fluor mais avec la condition que si c'est un atome d'hydrogène il soit à la position β par rapport au groupe sulfonique), ou de l'hydrate de cet acide, dans une proportion d'environ 0,1 à 20 mol-% par rapport à l'acide nitrobenzoïque, à des températures d'environ 60 à 120°C.

2. Procédé selon la révendication 1 caractérisé en ce que l'on effectue la réaction à des températures d'environ 62 à 108°C.

3. Procédé selon la revendication 1 ou 2, caractérisé en ce que l'on effectue la réaction en présence d'acide tétrafluoréthane-sulfonique ou d'acide hexafluoro-propane-sulfonique ou de leurs hydrates.

4. Procédé selon une ou plusieurs des revendications 1 à 3, caractérisé en ce que l'on effectue la réaction avec un excès d'environ 400 à 500 mol-% de l'alcanol en C₁-C₃.

5. Procédé selon une ou plusieurs des revendications 1 à 4, caractérisé en ce que l'on opère en présence d'environ 2 à 10 mol-% d'un acide polyfluoralcane-sulfonique de formule 1 selon la revendication 1 ou de son hydrate.

6. Procédé selon une ou plusieurs des revendications 1 à 5, caractérisé en ce que l'on opère avec l'acide 2-nitro-, 3-nitro- ou 4-nitrobenzoïque.

7. Procédé selon une ou plusieurs des revendications 1 à 6, caractérisé en ce que le solvant inerte employé est le benzène, le toluène, un xylène, le chlorobenzène, l'éthylbenzène, le tétrachlorure de carbone, le chloroforme, le cyclohexane, le 1,2-dichloréthane, l'éther isopropylique, l'éther méthylbutylique, la méthylbutylcétone, la méthyléthylcétone ou encore l'éther propylisopropylique.

8. Procédé selon une ou plusieurs des revendications 1 à 7, caractérisé en ce que l'on effectue la réaction sous pression réduite.

9. Procédé selon une ou plusieurs des revendications 1 à 7, caractérisé en ce que l'on effectue la réaction sous pression.

10. Procédé selon une ou plusieurs des revendications 1 à 9, caractérisé en ce que l'on ajoute l'alcanol en C₁-C₃ en excès à l'acide nitrobenzoïque en solution dans le solvant inerte préalablement placé dans le récipient de réaction, solution qui contient aussi le catalyseur, à des températures d'environ 62 à 108°C.
